# FASCICULE DE BREVET EUROPEEN

(11) **EP 2 858 684 B1**
(45) Date de publication et mention de la délivrance du brevet: **25.04.2018**
(21) Numéro de dépôt: 13733355.5
(22) Date de dépôt: 06.06.2013
(51) Int. Cl.: A61L 2/10, A61L 2/08, A61L 9/20, B05B 11/00, B05B 15/02

(54) **DISTRIBUTEUR DE PRODUIT FLUIDE**
FLÜSSIGPRODUKTSPENDER
FLUID PRODUCT DISPENSER

(30) Priorité: 07.06.2012 FR 1255311
(43) Date de publication de la demande: 15.04.2015
(73) Titulaire: APTAR France SAS, 27110 Le Neubourg (FR)
(72) Inventeur: DUQUET, Frédéric, F-78121 Crespières (FR); LÉOMENT, Simon, F-75017 Paris (FR)
(74) Mandataire: CAPRI
(86) Numéro de dépôt international: PCT/FR2013/051290
(87) Numéro de publication internationale: WO 2013/182815

(56) Documents cités:
- WO-A1-2007/018170
- WO-A1-2010/060979
- WO-A1-2010/103227
- WO-A1-2013/014345

## Description

La présente invention concerne un distributeur de produit fluide comprenant un organe de distribution, tel qu'une pompe, ayant un orifice de distribution de produit fluide au niveau duquel un utilisateur peut recueillir le produit fluide distribué. Le distributeur peut également comprendre un capot de protection amovible qui masque l'orifice de distribution en condition de stockage, le capot devant être retiré pour recueillir le produit fluide distribué à travers l'orifice de distribution. Les domaines d'application privilégiés de la présente invention sont ceux de la cosmétique, de la pharmacie ou encore de la parfumerie.

Des distributeurs de ce type sont très fréquemment utilisés dans le domaine de la cosmétique pour la distribution de produits visqueux, tels que des crèmes, des gels, etc. Le produit fluide sort de l'orifice de distribution sous la forme d'un cordon ou d'une noisette. L'utilisateur peut recueillir le produit fluide à l'aide d'un doigt ou sur une autre surface d'application souhaitée. Dans le cas de la présente invention, l'orifice de distribution désigne tout type d'ouverture terminale au niveau de laquelle le produit fluide est accessible par l'utilisateur. L'orifice de distribution peut notamment être formé au niveau d'un poussoir qui est déplaçable axialement en va-et-vient par l'utilisateur à l'aide d'un ou de plusieurs doigt(s). L'orifice de distribution s'étend souvent latéralement ou radialement par rapport à l'axe de déplacement du poussoir. Ainsi, lorsque l'on appuie sur le poussoir, l'orifice de distribution se déplace axialement sur une distance correspondant à la course du poussoir. Afin d'empêcher tout actionnement involontaire ou intempestif du poussoir, et de protéger l'orifice de distribution, le poussoir peut être coiffé ou masqué par un capot de protection qui se présente en général sous la forme d'un godet renversé. L'utilisateur peut appuyer sur le poussoir de manière à le déplacer axialement, ce qui engendre la distribution de produit fluide à travers l'orifice de distribution. Bien entendu, l'utilisateur va chercher à recueillir la totalité du produit fluide distribué au niveau de l'orifice de distribution. Toutefois, il subsiste toujours un résidu de produit fluide dans l'orifice de distribution et/ou autour de l'orifice de distribution. Etant donné qu'il est extrêmement difficile de recueillir ce résidu de produit fluide, l'utilisateur laisse le distributeur dans cet état. L'orifice de distribution peut ainsi être souillé, mais également d'autres surfaces du poussoir et du distributeur dans son ensemble. De ce fait, ce résidu de produit fluide va dessécher et se détériorer, engendrant l'apparition de micro-organismes, germes, bactéries, microbes, etc. A la prochaine distribution, l'utilisateur va à nouveau appuyer sur le poussoir de manière à distribuer du produit fluide à travers l'orifice de distribution. La nouvelle dose de produit fluide va bien entendu entrer en contact avec le résidu desséché et détérioré de produit fluide provenant de la distribution précédente. Il s'ensuit que la nouvelle dose de produit fluide va elle aussi être contaminée par les micro-organismes, germes, bactéries, microbes, etc. provenant du résidu de produit fluide. L'hygiène du produit fluide distribué n'est donc pas assurée.

Ce qui est valable pour un poussoir pourvu d'un capot ou non l'est également pour d'autres types de distributeurs, comme des pots, où l'utilisateur va aussi recueillir le produit fluide au niveau d'un orifice de distribution.

Dans un domaine connexe, on connaît les documents WO2007/018170 et WO2010/060979 qui prévoit de stériliser de la vapeur d'eau à l'aide de TiO2 irradié avec une lampe.

La présente invention a pour but de remédier à l'inconvénient précité de l'art antérieur en définissant un distributeur dont les doses successives distribuées ne sont pas contaminées par des résidus de produit fluide provenant de distributions précédentes. Un autre but de la présente invention est de ne pas bouleverser fondamentalement l'architecture du distributeur. Encore un autre but de l'invention est de stériliser ou de neutraliser les résidus de produit fluide sans manipulation autre que celles conventionnelles, comme par exemple le retrait et la remise en place du capot sur l'orifice de distribution.

Pour atteindre ces différents buts, la présente invention propose un distributeur de produit fluide tel que défini dans la revendication 1. Ainsi, le rayonnement issu du capot de protection a pour fonction d'activer ou de stimuler l'effet ou les propriétés bactéricides d'une substance photosensible qui va à son tour agir sur le produit fluide présent sur les surfaces de contact du distributeur pour les stériliser ou décontaminer. La surface de contact peut être choisie parmi un orifice de distribution, un poussoir, une coupelle de récupération, un conduit de distribution, etc. L'organe de distribution comprend un orifice de distribution de produit fluide par lequel le produit fluide sort du distributeur de manière à être accessible pour un utilisateur, le distributeur comprenant également un capot de protection amovible qui masque l'orifice de distribution en condition de stockage, la surface de contact étant définie au niveau de l'orifice de distribution, le capot étant pourvu de la source de rayonnement. L'orifice de distribution peut être formé dans une coupelle de récupération, la surface de contact s'étendant au niveau de cette coupelle. L'orifice de distribution peut être formé par un poussoir, la surface de contact s'étendant au niveau de ce poussoir.

Avantageusement, la source de rayonnement est disposée à proximité directe de la surface de contact.

Selon un autre aspect de l'invention, la source de rayonnement intègre des moyens de déclenchement du rayonnement et des moyens de temporisation aptes à couper le rayonnement au bout d'un laps de temps déterminé.

La source de rayonnement peut émettre un rayonnement, avantageusement émis par une diode électroluminescente LED, ayant une longueur d'onde de l'ordre de 280 à 380 nanomètres, pour activer l'effet bactéricide du photocatalyseur. Le photocatalyseur bactéricide peut être du dioxyde de titane TiO2. Le photocatalyseur bactéricide est appliqué sur la surface contact ou est intégré dans une paroi formant la surface de contact.

Un des principes de la présente invention est de se servir d'un rayonnement approprié pour irradier un photocatalyseur bactéricide qui va stériliser ou décontaminer les éventuels résidus de produit fluide présents sur les surfaces de contact du distributeur. Ce n'empêche toutefois pas le rayonnement de stériliser ou décontaminer directement les éventuels résidus de produit fluide.

L'invention sera maintenant plus amplement décrite en référence aux dessins joints donnant à titre d'exemple non limitatif un mode de réalisation de l'invention.

Sur les figures :
La figure 1 est une vue explosée en perspective d'un distributeur selon une forme de réalisation non limitative de l'invention,
La figure 2 est une vue en coupe transversale verticale à travers le distributeur de la figure 1,
La figure 3 est une vue en coupe transversale horizontale à travers le distributeur des figures 1 et 2 au niveau de l'orifice de distribution, et
La figure 4 est une vue en coupe transversale verticale à travers un ensemble de distribution selon un deuxième mode de réalisation de l'invention, et
La figure 5 est une vue en coupe transversale verticale à travers un distributeur selon un troisième mode de réalisation de l'invention.

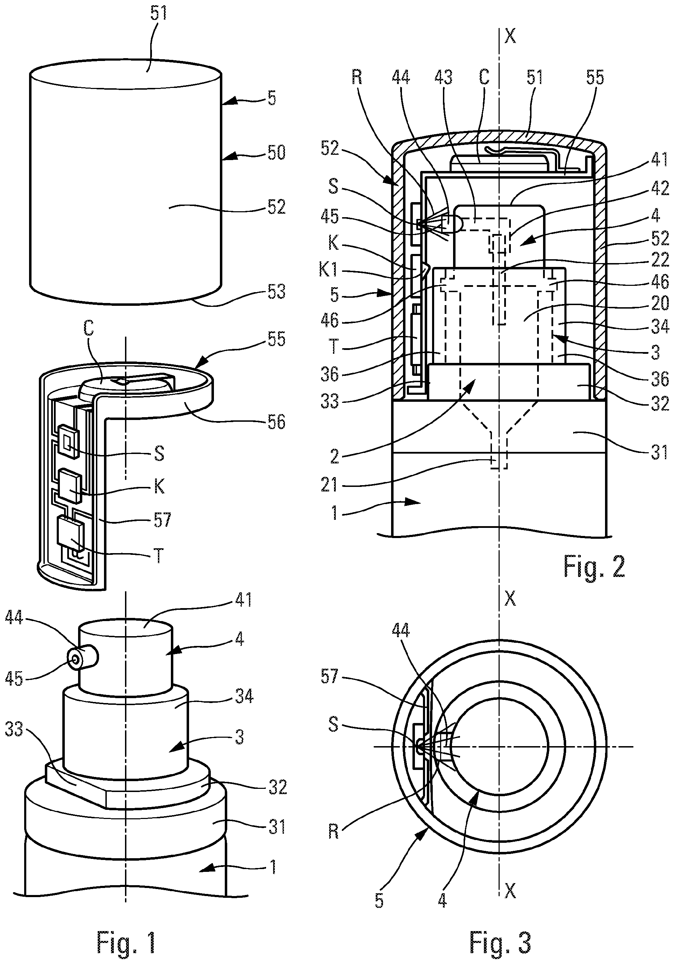

Le distributeur de produit fluide représenté sur les figures 1 à 3 est plus particulièrement destiné au produit fluide visqueux, comme des crèmes, des gels, etc. Il comprend essentiellement cinq éléments constitutifs, à savoir un réservoir 1, un organe de distribution 2 qui est une pompe, une bague de fixation 3 pour fixer la pompe sur le réservoir, un poussoir 4 monté sur la pompe et un capot 5 qui incorpore la présente invention ; à l'exception du capot 5 et dans une moindre mesure la bague de fixation 3, les autres éléments constitutifs, à savoir le réservoir 1, la pompe 2 et le poussoir 4 peuvent être de conception tout à fait conventionnelle.

Le réservoir de produit fluide 1 peut être de toutes natures, de toutes formes et réalisé en n'importe quel matériau approprié. Il définit un volume interne de stockage de produit fluide qui peut être constant ou variable. Dans le domaine de la cosmétique, il est plutôt d'usage d'utiliser des réservoirs de capacité variable, afin que le produit fluide stocké à l'intérieur ne rentre pas en contact avec l'air extérieur. Le réservoir comprend bien entendu une ouverture qui fait communiquer son volume interne avec l'extérieur.

L'organe de distribution 2, comme précité, est une pompe manuelle comprenant un corps de pompe 20 définissant une entrée 21 en communication avec le réservoir 1. La pompe 2 comprend également une tige d'actionnement 22 qui est déplaçable axialement en va-et-vient à l'intérieur du corps 20 de manière à faire varier le volume d'une chambre de pompe pour mettre une dose de produit fluide sous pression. Sur la figure 2, on peut voir que la tige d'actionnement 22 s'étend le long de la direction axiale X, qui constitue également un axe de symétrie, voire de révolution, pour le distributeur. Bien que non représentée, la tige d'actionnement 22 est pourvue d'un piston adapté à coulisser de manière étanche à l'intérieur d'un fût de coulissement de la chambre de pompe. Le fonctionnement de cette pompe est tout à fait conventionnel : en enfonçant la tige d'actionnement 22 dans le corps de pompe 20, le produit fluide contenu dans la chambre de pompe est mis sous pression de manière à être refoulé à travers la tige d'actionnement. Lorsque l'on relâche la tige d'actionnement 22 qui regagne sa position de repos sous l'action d'un ressort de rappel, du produit fluide en provenance du réservoir est aspiré dans la chambre de pompe à travers l'entrée 21.

La bague de fixation 3 a pour fonction principale de maintenir la pompe 2 par rapport au réservoir 1. La fixation est de préférence définitive et étanche. La bague de fixation 3 comprend une section basse 31 en prise avec l'ouverture du réservoir 1, une section intermédiaire 32 et une section supérieure 34. On peut remarquer sur la figure 1 que la section intermédiaire 32 comporte un méplat 33 qui rompt la circularité de la géométrie. Ce méplat 33, comme on le verra ci-après, va servir à l'indexation du capot 5 par rapport au reste du distributeur. La pompe 2 est maintenue par n'importe quel moyen approprié de manière fixe et étanche à l'intérieur de la bague de fixation 3. On peut remarquer sur la figure 1 que le corps de pompe s'étend à travers les trois sections de la bague de fixation. La tige d'actionnement 22 peut faire saillie axialement vers le haut hors de la bague de fixation 3.

Le poussoir 4 est monté sur l'extrémité libre de la tige d'actionnement 22, et peut être déplacé en va-et-vient selon la direction axiale X. De cette manière, le poussoir 4 entraîne la tige d'actionnement 22 dans le corps de pompe 20. Le poussoir 4 comprend un manchon de raccordement 42 en prise autour de l'extrémité libre de la tige d'actionnement 22, ce manchon communiquant avec un embout 44 par un conduit d'alimentation interne 43. L'embout 44 forme un orifice de distribution 45 visible sur la figure 1. On peut remarquer que l'embout 44 fait saillie radialement ou latéralement vers l'extérieur, de sorte que l'orifice de distribution 45 est orienté transversalement, et plus particulièrement perpendiculairement, par rapport à la direction axiale X. Ainsi, lorsque l'on déplace le poussoir 4 axialement en va-et-vient, l'orifice de distribution 45 se déplace également de manière solidaire avec le poussoir 4. Lors de son déplacement, le poussoir 4 pénètre partiellement à l'intérieur de la section supérieure 34 de la bague de fixation 3. Pour actionner le poussoir, ce dernier définit une surface d'appui 41 sur laquelle l'utilisateur peut appuyer à d'un doigt. Le poussoir comprend également une paroi latérale sensiblement cylindrique au niveau de laquelle est formé l'embout 44.

Au moins une partie du poussoir 4 définit des surfaces susceptibles de venir en contact avec du produit fluide : il s'agit notamment de l'orifice de distribution 45, de l'embout 44, de la surface d'appui et de la paroi latérale. Selon l'invention, ces surfaces de contact comprennent un photocatalyseur bactéricide : celui-ci peut être appliqué en surface ou intégré dans une paroi qui forme les surfaces de contact. Le photocatalyseur bactéricide peut être du TiO2. Le TiO2 réagit à un rayonnement ayant une longueur d'onde de l'ordre de 280 à 380 nanomètres qui amplifie ou active l'effet bactéricide du photocatalyseur.

Le capot de protection 5 comprend un corps de capot 50 qui est de préférence opaque. Ce corps de capot 50 présente une configuration générale en forme de godet renversé, définissant ainsi une paroi supérieure 51 et une paroi latérale sensiblement cylindrique 52 qui définit un bord inférieur annulaire 53. Une fois en place sur le distributeur, comme représenté sur la figure 2, la paroi supérieure 51 est disposée au-dessus du poussoir 4 et la paroi latérale 52 s'étend autour du poussoir 4 et de la bague de fixation 3. Plus précisément, la paroi latérale 52 entoure la section intermédiaire 32 et la section supérieure 34 de la bague de fixation : le bord annulaire inférieur 53 du capot 5 venant en appui sur la section inférieure 31 de la bague de fixation 3. On peut prévoir un encliquetage entre le capot 5 et la bague de fixation 3 pour stabiliser le maintien du capot 5 sur le distributeur en condition de stockage.

Le distributeur qui vient d'être décrit présente une conception tout à fait conventionnelle dans les domaines de la cosmétique, de la parfumerie ou encore de la pharmacie. Le poussoir 4, et plus particulièrement son orifice de distribution 45, associé au capot 5 constituent une tête de distribution de produit fluide au sens le plus large. Sans sortir du cadre de l'invention, il est possible de dissocier l'orifice de distribution du poussoir 4, par exemple de manière à rendre l'orifice fixe par rapport au réservoir 1. L'orifice de distribution 45 peut alors être relié au poussoir 4 par un conduit souple. D'autres configurations de distributeurs permettent également de dissocier l'orifice de distribution du poussoir 4. Dans le cadre de la présente invention, la tête de distribution doit être comprise comme l'association d'un orifice de distribution avec un capot de protection. Dans le cas particulier non limitatif des figures, la tête de distribution est constituée par le poussoir 4 et le capot 5.

Selon l'invention, le capot de protection 5 comprend un élément de support 55 qui est disposé à l'intérieur de l'espace formé par le capot. L'élément de support 55 peut par exemple former un plateau supérieur 56 et une languette latérale 57 reliés ensemble au niveau d'un bord du plateau de sorte que la languette 57 s'étend axialement vers le bas. Ainsi, cet élément de support 55 peut être inséré à l'intérieur du corps de capot 50 de sorte que le plateau 56 vienne se positionner juste sous la paroi supérieure 51 et la languette 57 juste contre la paroi latérale 52. Ceci est clairement visible sur la figure 2, et également compréhensible à partir de la figure 1. L'extrémité inférieure de la languette 57 s'étend sensiblement jusqu'au niveau de l'extrémité inférieure de la paroi latérale 52 du capot 5 de manière à pouvoir se positionner contre le méplat 33 de la section intermédiaire 32 de la bague de fixation 3. Ainsi, l'élément de support 55 impose l'orientation angulaire du capot de protection 5 par rapport à la bague de fixation 3, et de ce fait par rapport au restant du distributeur. Le capot 5 est donc toujours orienté de la même façon par rapport au distributeur. D'autre part, le poussoir 4 est bloqué en rotation par rapport au restant du distributeur de sorte que l'embout 44 et son orifice de distribution 45 sont toujours orientés de la même manière. Pour réaliser le blocage du poussoir 4, on peut par exemple prévoir deux ergots de guidage 46 au niveau du poussoir 4 qui coulissent dans deux rainures axiales 36 formées par la section supérieure 34 de la bague de fixation 3. Il s'agit là d'une caractéristique conventionnelle fréquemment utilisée pour bloquer le poussoir en rotation. Ainsi, le capot 5 est indexé par rapport au distributeur et l'orifice de distribution 45 est bloqué en rotation, ce qui impose le positionnement relatif de l'orifice de distribution 45 et du capot 5. Comme on peut le voir sur les figures 2 et 3, l'embout de distribution 44 est orienté vers la languette 57 dont l'extrémité inférieure vient en contact du méplat 33. Cela constitue le seul positionnement possible du capot 5 par rapport au distributeur et l'orifice de distribution.

Selon l'invention, l'élément de support 55 supporte une source de rayonnement S apte à émettre un rayonnement R destiné à irradier les surfaces de contact du poussoir susceptibles de venir en contact avec du produit fluide distribué. De cette manière, si du produit fluide résiduel subsiste par exemple au niveau de l'orifice de distribution et/ou autour de l'orifice de distribution, il sera décontaminé et/ou stérilisé par le photocatalyseur bactéricide dont l'effet bactéricide aura été catalysé ou activé par le rayonnement R. On peut voir sur les figures 2 et 3 que la source S et son rayonnement R sont disposés à proximité, et juste en face, de l'orifice de distribution 45. La source S est disposée sur une plaque de circuit imprimée montée sur la languette 57. Pour l'activation de la source S, il est prévu des moyens de déclenchement K par exemple sous la forme d'un interrupteur comprenant un organe de déclenchement K1 qui peut être mécanique et/ou électronique. On peut par exemple prévoir que l'organe de déclenchement K1 vienne en contact avec la section supérieure 34 de la bague de fixation 3. On peut également prévoir l'organe de déclenchement K1 sous la forme d'un détecteur de présence qui détecte la présence d'un objet à l'intérieur du capot. L'organe de déclenchement K1 peut ainsi fonctionner avec ou sans contact direct. Les moyens de déclenchement K sont avantageusement associés à des moyens de temporisation T aptes à couper le rayonnement R au bout d'un laps de temps déterminé. Les moyens de temporisation peuvent par exemple agir sur les moyens de déclenchement K pour les réinitialiser. On peut par exemple prévoir une durée de rayonnement de l'ordre de 10 secondes à 1 minute. Et pour l'alimentation électrique du système, il est prévu une batterie C qui peut être disposée au niveau du plateau 56. Une fois l'élément de support 55 ainsi équipé, il est inséré dans le corps de capot 50 avec tous les éléments électroniques disposés entre l'élément de support 55 et le corps de capot, de sorte qu'aucun élément électronique n'est visible.

La manipulation de ce capot de protection 5 est strictement le même qu'un capot de protection classique. Il est mis en place et retiré en le déplaçant de manière axiale dans la direction X. Lorsque l'on le met en place, l'organe de déclenchement K1 détecte la présence du poussoir 4 et/ou de la bague de fixation 3, ou en variante vient en contact direct avec le poussoir 4 et/ou la bague de fixation 3, de manière à déclencher le rayonnement R de la source S. Au cours de cette opération, l'utilisateur n'intervient aucunement sur le déclenchement et le fonctionnement de la source S. Le rayonnement R est ainsi émis pendant un laps de temps déterminé par les moyens de temporisation T. Au terme de ce laps de temps, le rayonnement R est arrêté. Le capot de protection 5 remplit alors à nouveau une simple fonction classique de protection. Lorsque l'utilisateur retire axialement le capot 5, la source S reste inactive : ce n'est que lors de la remise en place du capot sur le distributeur que la source va à nouveau émettre son rayonnement pendant un laps de temps déterminé. Par conséquent, la gestuelle de déplacement du capot est tout à fait conventionnelle. L'utilisateur peut même ignorer la présence de la source de rayonnement et de ces composants électroniques associés.

Selon un mode d'application de la présente invention, le rayonnement R est un rayonnement ayant une longueur d'onde de l'ordre de 280-380 nanomètres, apte à remplir une fonction de catalyse pour le photocatalyseur bactéricide, qui peut être du TiO2. Le rayonnement peut par exemple être émis par une diode électroluminescente LED. Le rayonnement peut également participer à la destruction directe des bactéries présentes dans le produit fluide. L'intensité est bien entendu dépendante de la puissance de la source S, de la distance source/orifice et du temps d'irradiation du rayonnement R. Les résidus de produit fluide qui s'accumulent au niveau de l'orifice de distribution, et/ou autour de l'embout de distribution 44, sont ainsi décontaminés et/ou stérilisés, de sorte que la dose de produit fluide distribuée ultérieurement ne sera pas contaminée par les résidus de produit fluide provenant des distributions précédentes.

Dans le mode de réalisation qui vient d'être décrit, la source de rayonnement S est embarquée dans le capot 5. En variante non représentée, on peut également envisager de déporter cette source dans un autre élément constitutif du distributeur, comme par exemple la bague de fixation 3 et d'acheminer le rayonnement au moyen d'un guide d'onde ou d'une fibre optique au niveau de l'orifice de distribution. Il en est de même pour les éléments électroniques associés comme les moyens de déclenchement K et les moyens de temporisation T, ainsi que pour la batterie C, qui pourraient être logés à l'intérieur de la bague de fixation 3. Un principe de l'invention est de se servir du capot 5 comme support d'un rayonnement émis directement sur l'orifice de distribution et son environnement proche. Un autre principe est de se servir de ce rayonnement pour activer ou amplifier l'effet bactéricide d'un photocalatyseur bactéricide.

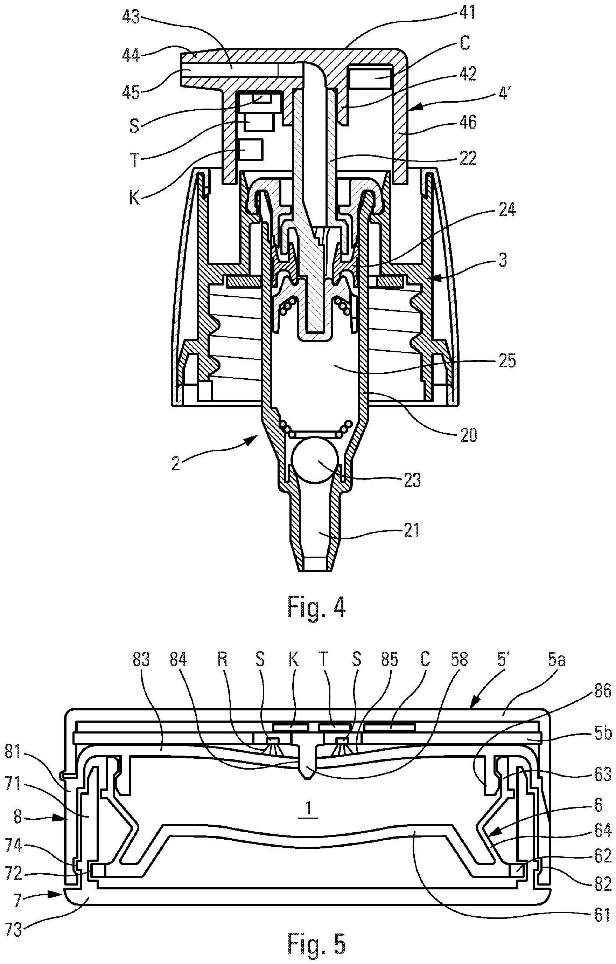

La figure 4 montre un ensemble de distribution prêt à être monté sur un réservoir pour ainsi constituer un distributeur de produit fluide. Cet ensemble comprend une pompe 2 ayant un corps de pompe 20, une entrée 21, une tige d'actionnement 22, un clapet d'entrée 23 et un piston 24, définissant ensemble une chambre de pompe classique. La pompe est pourvue d'une bague de fixation 3 et d'un poussoir 4'. Tout comme dans le premier mode de réalisation, le poussoir est monté sur l'extrémité libre de la tige d'actionnement 22, et peut être déplacé en va-et-vient selon la direction axiale X. De cette manière, le poussoir 4 entraîne la tige d'actionnement 22 dans le corps de pompe 20. Le poussoir 4 comprend un manchon de raccordement 42 en prise autour de l'extrémité libre de la tige d'actionnement 22, ce manchon communiquant avec un embout 44 par un conduit d'alimentation interne 43. L'embout 44 forme un orifice de distribution 45 visible sur la figure 1. On peut remarquer que l'embout 44 fait saillie radialement ou latéralement vers l'extérieur, de sorte que l'orifice de distribution 45 est orienté transversalement, et plus particulièrement perpendiculairement, par rapport à la direction axiale X. Pour actionner le poussoir, ce dernier définit une surface d'appui 41 sur laquelle l'utilisateur peut appuyer à d'un doigt. Le poussoir comprend également une paroi latérale sensiblement cylindrique 46 au niveau de laquelle est formé l'embout 44.

Selon l'invention, le poussoir 4' contient au moins une source S de rayonnement R, une batterie C, des moyens de déclenchement K du rayonnement et des moyens de temporisation T aptes à couper le rayonnement R au bout d'un laps de temps déterminé, tout comme dans le mode de réalisation précédents. Tous ces éléments peuvent être logés à l'intérieur du poussoir. Avantageusement, la source S émet son rayonnement R directement en direction du conduit 43 et de la surface d'appui 41. Avantageusement, la matière constitutive du poussoir est au moins localement transparente au rayonnement R, de sorte qu'il puisse diffuser à travers la masse du poussoir. D'autre part, la matière constitutive du poussoir contient un photocalatyseur bactéricide, comme du TiO2, qui appliqué en surface, ou de préférence noyé dans la masse. Le rayonnement, d'une longueur d'onde de l'ordre de 280-380 nanomètres, active ou amplifie l'effet bactéricide du photocalatyseur qui va agir sur les résidus de produit fluide présents sur le poussoir, notamment au niveau de l'orifice distribution 45, de l'embout 44, mais également au niveau de la surface d'appui 41 ou de la paroi latérale 46.

Dans ce deuxième mode de réalisation, on sert du poussoir pour diffuser le rayonnement catalyseur du photocatalyseur bactéricide. Un tel poussoir diffuseur peut également être utilisé dans le premier mode de réalisation.

En se référant maintenant à la figure 5, on voit un distributeur de produit fluide sous la forme d'un pot qui comprend essentiellement quatre éléments constitutifs, à savoir un soufflet 6, des moyens d'actionnement 7, une coque 8 et un capot ou couvercle de protection 5'.

Le soufflet 6 est une pièce monobloc par exemple réalisée dans un matériau souple déformable tel qu'un élastomère ou un thermoplastique élastomère. Il présente de bonnes propriétés de déformabilité dans des zones bien précises. Le soufflet 1 comprend une paroi de fond 61 sensiblement rigide. Sur sa périphérie extérieure, la paroi de fond 11 définit plusieurs brides de déplacement 62 sensiblement rigides. Le soufflet 6 forme également un collier de fixation 63 qui est également sensiblement rigide. Le soufflet 1 comprend également une paroi déformable 64 qui relie le collier de fixation 63 à la paroi de fond 61. Ainsi, il est possible de rapprocher translativement la paroi 61 du collier de fixation 15 par déformation de la paroi 64. Plus précisément, la paroi déformable 64 forme un pli adapté à s'accentuer si l'on exerce une pression tendant à rapprocher le collier 63 des brides 62. C'est précisément cette paroi déformable 44 qui donne le nom à la pièce puisqu'elle lui confère une fonction de soufflet. Le soufflet 6 peut par exemple être remplacé par une poche souple fixée sur un support de poche remplissant la fonction du collier de fixation 63. La paroi de fond 61 peut être remplacée par une plaque sur laquelle vient reposer la poche souple.

Les moyens d'actionnement 7 comprennent une douille filetée intérieurement 71 de manière à former un ou plusieurs filets ou filetages internes 72. Comme on peut le voir sur la figure 5, les brides de déplacement 62 du soufflet sont en prise filetée avec ces filetages internes 72. En dessous de la douille filetée 71, les moyens d'actionnement définissent un fond 73 qui sert d'élément d'actionnement et de préhension que l'utilisateur peut saisir pour entraîner les moyens d'actionnement en rotation. Sur la paroi externe de la douille filetée 20, il est formé un cordon continu ou plusieurs segments de cordon saillants 74 qui s'étendent sur une partie ou la totalité de la périphérie de la douille 71. On comprend aisément qu'une rotation des moyens d'actionnement 7, alors que le soufflet 6 est maintenu fixement, entraîne un déplacement des brides 62 à l'intérieur de la douille filetée 71.

Le corps 8 du distributeur forme une sorte de coque extérieure qui confère en grande partie l'aspect esthétique au distributeur. Le corps 8 comprend une jupe périphérique 81 à l'intérieur de laquelle est disposée la douille 71 des moyens d'actionnement 7. La jupe 81 est formée intérieurement avec un logement annulaire 82 dans lequel sont reçus les segments de cordon saillants 74 formés à l'extérieur de la douille filetée 71. L'engagement des segments de cordon 74 dans le logement 82 assure le maintien rotatif de la douille 71 dans la jupe 81. Ainsi, les moyens d'actionnement 7 peuvent tourner librement dans la jupe 81, mais ne peuvent pas s'en dégager. Au-dessus de la jupe 81, le corps 8 forme un plateau 83 dans lequel est formé un trou qui sert d'orifice de distribution 84 pour le distributeur. L'orifice 84 est avantageusement situé dans une coupelle de récupération 85 formé par le plateau 83. Un manchon de raccordement 86 s'étend sous le plateau 83 et est en prise avec le collier de fixation étanche 63 du soufflet 6. Ainsi, le soufflet 6 est relié de manière fixe même en rotation avec le corps 8. De ce fait, le soufflet 6 et le corps 8 forment ensemble un réservoir de volume variable étant donné que la paroi mobile 61 peut se déplacer en direction du plateau 83 par déformation de la paroi 64. L'utilisateur peut par exemple se saisir du corps 8 à l'aide de la main gauche et faire tourner les moyens d'actionnement 7 de la main droite en les saisissant au niveau de son fond 73. Ceci a pour effet de faire remonter les brides de déplacement 62 dans les filetages internes de la douille 72. La paroi mobile 61 remonte alors vers le plateau 83 en réduisant le volume utile du réservoir 1. Le produit fluide stocké à l'intérieur du réservoir est alors refoulé à travers l'orifice de distribution 84 de manière à pouvoir être collecté par l'utilisatrice dans la coupelle de récupération 85.

Le couvercle ou capot 5' peut être relié de manière monobloc par un pont de matière au corps 8. Le couvercle 5' comprend une coupelle 5a et une plaque 5b rapportée dans la coupelle pour former entre elles un logement contenant au moins une source S de rayonnement R, une batterie C, des moyens de déclenchement K du rayonnement et des moyens de temporisation T aptes à couper le rayonnement R au bout d'un laps de temps déterminé, tout comme dans les modes de réalisation des figures précédentes. La plaque 5b comprend autant d'ouvertures qu'il y a de sources de rayonnement R, de manière à diriger le rayonnement vers la coupelle 85 et l'orifice de distribution 84. La plaque 5b définit sur sa face inférieure un pointeau d'obturation 58 adapté à venir se loger de manière étanche dans l'orifice de distribution 84 pour l'obturer hermétiquement. Ceci est le cas lorsque le couvercle 5' est rabattu sur le plateau 83. Le rayonnement de la source S peut ainsi également irradier une partie du pointeau 58, et même une partie de la plaque 5b.

D'autre part, la coupelle 85, l'orifice 84, le pointeau 58 et/ou la plaque 5b, qui définissent tous des surfaces susceptibles de venir en contact avec du produit fluide, comprennent un photocatalyseur bactéricide qui est appliqué en surface ou intégré dans la masse. En tous cas, le rayonnement R de la ou des sources S irradie la surface de contact de cet ou ces éléments où le photocatalyseur bactéricide est présent, ce qui a pour effet d'activer l'effet bactéricide. Le photocatalyseur bactéricide peut être du TiO2, comme dans les exemples précédents.

Dans tous les modes de réalisation, le rayonnement de la source est utilisé pour activer, amplifier ou stimuler les propriétés bactéricides d'une substance sensible, comme le TiO2. Cette association d'un rayonnement approprié et d'un photocatalyseur bactéricide peut être appliquée à n'importe quelle surface d'un distributeur de produit fluide. Il n'est cependant pas exclu que le rayonnement ait une action bactéricide directe sur le produit fluide. Il faut toutefois noter que pour qu'une source d'irradiation ait un effet bactéricide direct, sa longueur d'onde doit être comprise entre 250 et 300 nanomètres (domaine des UVC), idéalement 254 nanomètres. Le coût de ces sources lumineuses reste élevé et leur rendement faible dans la frange 250 - 280 nanomètres.

L'invention consiste à utiliser une source avec une longueur d'onde plus grande (> 280 nanomètres) placée dans le domaine des UVB et UVA, et permettant d'activer l'effet bactéricide d'un adjuvant contenant dans la matière plastique, par exemple du poussoir. En l'occurrence l'adjuvant utilisé est du dioxyde de titane (TiO2). L'effet bactéricide du TiO2 est en effet augmenté s'il est soumis à une irradiation de longueur d'onde inférieure à 385 nanomètres.

La réalisation de sources lumineuses dans des longueurs d'onde comprise entre 280 et 380 nanomètres est moins couteuse et leur rendement plus élevé que celle réalisées dans des longueurs d'onde ayant un effet bactéricide direct (254 nanomètres).

## Revendications

1. Distributeur de produit fluide comprenant un réservoir (1) et un organe de distribution (2 ; 6, 7, 8) comprenant un orifice de distribution de produit fluide (45 ; 84) par lequel le produit fluide sort du distributeur de manière à être accessible pour un utilisateur, le distributeur comprenant au moins une surface de contact destinée à venir en contact avec le produit fluide, la surface de contact comprenant un photocatalyseur bactéricide, le distributeur comprenant en outre une source (S) de rayonnement (R) pour irradier le photocatalyseur bactéricide, le distributeur comprenant un capot de protection amovible (5 ; 5') qui masque l'orifice de distribution en condition de stockage,
**caractérisé en ce que** la source (S) de rayonnement (R) est supportée par le capot de protection amovible (5 ; 5'), la surface de contact étant définie au niveau de l'orifice de distribution (45 ; 84).

2. Distributeur selon la revendication 1, dans lequel la surface de contact est choisie parmi :
- l'orifice de distribution (45 ; 84),
- un poussoir (4 ; 4'),
- une coupelle de récupération (85),
- un conduit de distribution (43).

3. Distributeur selon la revendication 2, dans lequel l'orifice de distribution (84) est formé dans une coupelle de récupération (85), la surface de contact s'étendant au niveau de cette coupelle (85).

4. Distributeur selon l'une quelconque des revendications précédentes, dans lequel l'orifice de distribution (45) est formé par un poussoir, la surface de contact s'étendant au niveau de ce poussoir.

5. Distributeur selon l'une quelconque des revendications précédentes, dans lequel la source de rayonnement (R) est disposée à proximité directe de la surface de contact.

6. Distributeur selon l'une quelconque des revendications précédentes, dans laquelle la source de rayonnement (R) intègre des moyens de déclenchement (K) du rayonnement (R) et des moyens de temporisation (T) aptes à couper le rayonnement (R) au bout d'un laps de temps déterminé.

7. Distributeur selon l'une quelconque des revendications précédentes, dans laquelle la source de rayonnement (S) émet un rayonnement (R), avantageusement émis par une diode électroluminescente LED, ayant une longueur d'onde de l'ordre de 280 à 380 nanomètres, pour activer l'effet bactéricide du photocatalyseur.

8. Distributeur selon l'une quelconque des revendications précédentes, dans laquelle le photocatalyseur bactéricide est du dioxyde de titane TiO2.

9. Distributeur selon l'une quelconque des revendications précédentes, dans laquelle le photocatalyseur bactéricide est appliqué sur la surface contact ou est intégré dans une paroi formant la surface de contact.

## Patentansprüche

1. Flüssigproduktspender mit einem Vorratsbehälter (1) und einem Abgabeelement (2; 6, 7, 8) mit einer Flüssigprodukt-Abgabeöffnung (45; 84), über die das Flüssigprodukt aus dem Spender auf eine Weise austritt, dass es für einen Benutzer zugänglich ist, wobei der Spender mindestens eine Kontaktfläche umfasst, die dazu bestimmt ist, dass sie mit dem Flüssigprodukt in Kontakt kommt, wobei die Kontaktfläche einen bakteriziden Fotokatalysator aufweist, wobei der Spender ferner eine Quelle (S) einer Strahlung (R) zum Bestrahlen des bakteriziden Fotokatalysators umfasst, wobei der Spender eine abnehmbare Schutzabdeckung (5; 5') umfasst, die im Lagerzustand die Spenderöffnung abdeckt,
**dadurch gekennzeichnet, dass** die Quelle (S) einer Strahlung (R) von der abnehmbaren Schutzabdeckung (5; 5') gehalten wird, wobei die Kontaktfläche im Bereich der Abgabeöffnung (45; 84) ausgebildet ist.

2. Spender nach Anspruch 1, bei dem die Kontaktfläche ausgewählt ist aus:
- der Abgabeöffnung (45; 84),
- einem Drücker (4; 4')
- einer Auffangschale (85),
- einer Abgabeleitung (43).

3. Spender nach Anspruch 2, bei dem die Abgabeöffnung (84) in einer Auffangschale (85) ausgebildet ist, wobei sich die Kontaktfläche im Bereich dieser Schale (85) erstreckt.

4. Spender nach einem der vorhergehenden Ansprüche, bei dem die Abgabeöffnung (45) durch einen Drücker gebildet ist, wobei sich die Kontaktfläche im Bereich dieses Drückers erstreckt.

5. Spender nach einem der vorhergehenden Ansprüche, bei dem die Quelle einer Strahlung (R) in unmittelbarer Nähe der Kontaktfläche angeordnet ist.

6. Spender nach einem der vorhergehenden Ansprüche, bei dem die Quelle einer Strahlung (R) Auslösemittel (K) für die Strahlung (R) beinhaltet sowie Zeitsteuermittel (T), die zum Abschalten der Strahlung (R) nach Ablauf einer vorbestimmten Zeitspanne ausgelegt sind.

7. Spender nach einem der vorhergehenden Ansprüche, bei dem die Strahlungsquelle (S) eine Strahlung (R) abgibt, die vorteilhafterweise von einer Leuchtdiode LED abgestrahlt wird, mit einer Wellenlänge einer Größenordnung von 280 nm bis 380 nm, um dadurch die bakterizide Wirkung des Fotokatalysators zu aktivieren.

8. Spender nach einem der vorhergehenden Ansprüche, bei dem der bakterizide Fotokatalysator aus Titandioxid TiO₂ besteht.

9. Spender nach einem der vorhergehenden Ansprüche, bei dem der bakterizide Fotokatalysator auf der Kontaktfläche aufgebracht ist, oder in eine Wand eingebracht ist, die die Kontaktfläche bildet.

## Claims

1. A fluid dispenser comprising a reservoir (1) and a dispenser member (2; 6, 7, 8) including a fluid dispenser orifice (45; 84) via which the fluid is dispensed from the dispenser so as to be accessible to a user, the dispenser including at least one contact surface for coming into contact with the fluid, the contact surface including a bactericidal photocatalyst, the dispenser further including a source (S) of radiation (R) for irradiating the bactericidal photocatalyst, the dispenser including a removable protective cap (5; 5') that masks the dispenser orifice in the storage condition;
**characterized in that** the source (S) of radiation (R) is supported by a removable protective cap (5; 5'), the contact surface being defined at the dispenser orifice (45; 84).

2. A dispenser according to claim 1, wherein the contact surface is selected from:
• the dispenser orifice (45; 84);
• a pusher (4; 4');
• a collection dish (85);
• a dispenser duct (43).

3. A dispenser according to claim 2, wherein the dispenser orifice (84) is formed in a collection dish (85), the contact surface extending in the dish (85).

4. A dispenser according to any preceding claim, wherein the dispenser orifice (45) is formed by a pusher, the contact surface extending over the pusher.

5. A dispenser according to any preceding claim, wherein the source of radiation (R) is arranged in the direct proximity of the contact surface.

6. A dispenser according to any preceding claim, wherein the source of radiation (R) incorporates trigger means (K) for triggering the radiation (R), and timer means (T) that are suitable for interrupting the radiation (R) at the end of a determined period of time.

7. A dispenser according to any preceding claim, wherein the radiation source (S) emits radiation (R), advantageously emitted by an LED, having a wavelength in the range about 280 nm to 380 nm, so as to activate the bactericidal effect of the photocatalyst.

8. A dispenser according to any preceding claim, wherein the bactericidal photocatalyst is titanium dioxide TiO₂.

9. A dispenser according to any preceding claim, wherein the bactericidal photocatalyst is applied to the contact surface or is incorporated in a wall forming the contact surface.
